# EUROPÄISCHE PATENTSCHRIFT

(11) **EP 2 329 262 B1**
(45) Veröffentlichungstag und Bekanntmachung des Hinweises auf die Patenterteilung: **21.03.2012**
(21) Anmeldenummer: 09759640.7
(22) Anmeldetag: 27.08.2009
(51) Int. Cl.: G01N 33/50, G01N 33/564, G01N 33/569, G01N 33/68, A61M 1/36, C12N 5/00, A61M 1/34

(54) **FILTERSYSTEM ZUR EXTRAKORPORALEN ABREICHERUNG VON AKTIVIERTEN POLYMORPHKERNIGEN GRANULOYZTEN (POLYMORPHONUCLEAR LEUKOCYTES - PMNS)**
FILTER SYSTEM FOR EXTRACORPOREAL DEPLETION OF ACTIVATED POLYMORPHONUCLEAR LEUKOCYTES (PMNS)
SYSTÈME DE FILTRAGE PERMETTANT L'ADMINISTRATION EXTRA-CORPORELLE DE GRANULOCYTES POLYMORPHONUCLÉAIRES (LEUCOCYTES POLYMORPHONUCLÉAIRES - PMN) ACTIVÉS

(30) Priorität: 01.09.2008 DE 102008045127
(43) Veröffentlichungstag der Anmeldung: 08.06.2011
(73) Patentinhaber: Heinrich, Hans-Werner, Prof. Dr., 17489 Greifswald (DE)
(72) Erfinder: Heinrich, Hans-Werner, Prof. Dr., 17489 Greifswald (DE)
(74) Vertreter: Baumbach, Friedrich
(86) Internationale Anmeldenummer: PCT/DE2009/001209
(87) Internationale Veröffentlichungsnummer: WO 2010/022714

(56) Entgegenhaltungen:
- WO-A2-02/24307
- SCHEFOLD JOERG C ET AL: "A novel selective extracorporeal intervention in sepsis: immunoadsorption of endotoxin, interleukin 6, and complement-activating product 5a." SHOCK (AUGUSTA, GA.) OCT 2007, Bd. 28, Nr. 4, Oktober 2007 (2007-10), Seiten 418-425, XP8118925 ISSN: 1073-2322 in der Anmeldung erwähnt
- NALESSO F: "Plasma filtration adsorption dialysis (PFAD): A new technology for blood purification" INTERNATIONAL JOURNAL OF ARTIFICIAL ORGANS, Bd. 28, Nr. 7, Juli 2005 (2005-07), Seiten 731-738, XP8119161 ISSN: 0391-3988
- TETTA C ET AL: "Extracorporeal treatments in sepsis: are there new perspectives?" CLINICAL NEPHROLOGY NOV 2003, Bd. 60, Nr. 5, November 2003 (2003-11), Seiten 299-304, XP8119194 ISSN: 0301-0430

## Beschreibung

Die Erfindung betrifft ein Filtersystem zur Entfernung von aktivierten PMNs und Mediatoren, die für deren Aktivierung verantwortlich sowie gegebenenfalls Mediatoren, die zeitgleich aber unabhängig von aktivierten PMNs für einen krankmachenden Zustand verantwortlich sind, aus Körperflüssigkeiten, Verfahren zu ihrer Herstellung und ihre Verwendung.
In den letzten Jahren hat sich das Wissen zur Pathophysiologie der systemischen Entzündung (Systemic Inflammatory Response Syndrom - SIRS) und multiplen Organversagen wesentlich verbessert. Zytokine spielen offenbar eine entscheidende Rolle in der Regulation wie Deregulation der systemischen Entzündungsreaktion. Aktivierten CD14+ Zellen produzieren Zytokine (TNFα, Interleukin-1 (IL-1), IL-6, IL-8), die via Zytokin-Rezeptor der Zielzelle ihre Wirkung ausüben.
Neben Zytokinen kommen immunkompetenten Lymphozyten und PMNs eine ursächliche Funktion in der Abwehr von Infektionen und dem Erhalt der Organintegrität nach Verletzungen/Operationen zu.
Parallel zur Stimulierung der Monozyten, Makrophagen, Lymphozyten und PMNs wird das Komplementsystem aktiviert. Es ist ein integrierter Bestandteil der immunologischen Abwehr der Säugetiere zur unmittelbaren und unspezifischen Bekämpfung bakterieller Mikroorganismen und Fremdpartikel. Von den im Blutserum vorkommenden Komplementproteinen, vorrangig Proenzyme, die durch proteolytische Spaltung aktiviert werden, besitzt das C3-Protein mit einer Serumkonzentration von ca. 1g/l eine zentrale Rolle. Nach Kontakt der Mikroorganismen mit dem C3 wird das Komplementprotein C3a abgespalten und durch das entstehende C3b wird einerseits die Bildung der C5-Konvertase eingeleitet (alternativer Weg der Komplementaktivierung) und andererseits die Reaktion dadurch amplifiziert, indem das C3b durch Anlagerung von Serumfaktoren sich zur C3-Konvertase wandelt. Das ebenfalls im Serum vorkommende Komplementprotein C5 wird durch die nun verstärkt bereitgestellte C5-Konvertase proteolytisch unter Bildung von C5a gespalten. An das entstandene C5b lagern sich weitere Komplementproteine (C6-C9) an, bis schlussendlich der polymere hydrophobe Membranangriffskomplex (MAK) gebildet wurde, der sich in die Bakterienmenbran (Opsonidierung) einlagert und Poren bildet, die zur Phagozytose und damit zur Elimination der Mikroorganismen (und des gebundenen MAK) führen. Die im Prozess der Komplementaktivierung freigesetzten Komplementfaktoren C3a und C5a (Anaphylatoxine) bewirken durch eine Erhöhung der Gefäßpermeabilität und durch die durch sie induzierte Freisetzung von Chemotoxinen das Anlocken der phygozytierenden Zellen an den Ort des bakteriellen Befalls. Die Verringerung der Anzahl an Bakterien bewirkt die Verminderung der Aktivierung des Komplementsystems. Diese unmittelbare und unspezifische Reaktion ist mit den anderen immunologischen Abwehrsystemen insofern eng verwoben, indem beispielsweise durch Komplementfaktoren die Synthese und Freisetzung der für die zelluläre Abwehr essentiellen Zytokine reguliert wird. Um die inflammatorische Wirkung zu vermitteln, werden C3a und C5a an spezifische zellständige Rezeptoren gebunden, die wiederum in Abhängigkeit von der Immunreaktivität unterschiedlich stark exprimiert werden. Um die Immunabwehr permanent reaktionsbereit zu erhalten, sind aktivierte Komplementfaktoren nicht nur nach Befall mit Mikroorganismen nachweisbar, sondern integrierter Bestandteil des Serums von Normalpersonen in einer Konzentration von 1 - 10 ng/ml.
Insbesondere bei einer entwickelten Sepsis, bei akutem Lungenversagen und bei moribunden Patienten können die Plasmaspiegel der Anaphylatoxine mehr als tausendfach erhöht sein.

Die lokale Anreicherung von Komplement kann verbunden sein mit schädigenden Auswirkungen für das Gewebe, so Herzinfarkt, zerebrale Malaria, Hirntraumen und ischämischen Zuständen anderer Genese.
PMNs und immunkompetente Lymphozyten sind wesentliche zellulare Komponenten für eine erfolgreiche Sofort- und Spätreaktion in der Abwehr von Infektionen und dem Erhalt der Organintegrität nach Verletzungen/Operationen.
Eine erhöhte Apoptose der Lymphozyten in der Spätphase der generalisierten Entzündung ist eine Überlebenschance verringernden Komplikation. Hotchkiss et al. (Accelerated lymphocyte death in sepsis occurs by both the death receptor and mitochondrial pathways. J Immunol 174: 5110-5118, 2005) zeigte, dass die Apoptose der immunkompetenten Lymphozyten in der hypo-inflammatorischen Phase eine weitere zentrale Rolle bei der Pathogenese der Sepsis einnimmt. Bei Patienten, die an einer Sepsis verstarben, konnten sehr hohe Apoptoseraten der Lymphozyten gemessen werden, wobei der Grad der Lymphozyten-Apoptose mit der Schwere der Sepsis korrelierte. Die Leukozytopenie beruht somit auf der erhöhten Apoptose hauptsächlich der CD4+ T-Zellen und B-Zellen. Aufgrund dieser Lymphopenie ist das Immunsystem nicht mehr in der Lage adäquat auf einen Erreger zu reagieren.
PMNs stellen gemeinsam mit dem Komplementsystem die erste Barriere des angeborenen Abwehrsystems dar. PMNs werden am Ort der Verletzung und/oder Eindringens von Mikroorganismen aktiviert im Ergebnis ihrer Stimulierung durch humorale pro-inflammatorische Mediatoren (TNF-alpha, IL-1ß, IL-6, IL-8, GM-CSF, Interferon-gamma). Diese Zytokine werden von verschieden Zellarten synthetisiert, einschließlich immunkompetenter Zellen. Eine überschießende Freisetzung dieser Zytokine führt zum SIRS, was letztlich Multiorganversagen zur Folge haben kann.
Die aktivierten PMNs phagozytieren die Mikroorganismen und setzen zytotoxische Substanzen wie Enzyme und aktiven Sauerstoff (H2O2) frei.
Die Lebenszeit der PMNs ist mit 6-8 Stunden sehr kurz. Die alternden PMNs unterliegen der Apoptose und werden letztlich von Makrophagen aufgenommen und abgebaut. Der gleiche Prozess läuft auch während einer lokalen Aktivierung ab. Die aktivierten PMNs sind ein wesentlicher Teil der lokalen Entzündung. Ist das Problem behoben, unterliegen die aktivierten PMNs der Apoptose und werden vom Makrophagen phagozytiert, ohne dass die im Inneren akkumulierten zytotoxischen Substanzen frei gesetzt werden. Mit dem Abklingen der lokalen Entzündung bewirken Mediatoren ein Gegensteuern der Aktivierung und die phagozytotische Beseitigung. So kann die nicht-selektive Zytotoxizität keine Schäden im umgebenden Gewebe anrichten.
Eine verzögerte Einleitung der Apoptose bei aktivierten PMNs ist also ein gewollter physiologischer Prozess zum Erhöhen der PMN-Funktion als Reaktion auf eine Infektion oder Verletzung. Eine persistierende pro-inflammatorische Hyperzytokinämie und das Einsetzten einer systemischen Entzündungsreaktion in Folge z.B. einer großflächigen Verletzung führt zur Hemmung der apoptotischen Clearance der aktivierten PMNs.

Diese nicht der Apoptose verfallenden PMNs stehen dem Organismus in der Abwehr einer Infektion zur Verfügung. Mit dem gewollten Effekt einer erhöhten Infektionsabwehr wächst allerding auch das Risiko, dass die zytotoxischen Substanzen der aktivierten PMNs in das umgebende Gewebe abgegeben werden.

So wurde in Blut von SIRS-, Sepsis-, ARDS-, Trauma- und Verbrennungspatienten eine verzögerte PMN-Apoptose festgestellt. Hirano et al. wiesen eine signifikante Korrelation zwischen hohen IL6-Spiegel und verzögerter PMN-Apoptose nach (Takeshi Hirano, T. et al. : Modulation of Polymorphonuclear Leukocyte Apoptosis in the Critically III by Removal of Cytokines with Continuous Hemodiafiltration, Blood Purif 2004;22:188-197). Mit der Höhe des IL6-Spiegels korrelierte auch die Schwere der systemischen Entzündung (APACHE II scores).

Mit der Schwere der systemischen Entzündung gehen auch Störungen in den PMN-Funktion einher. So haben Tavare-Murta et al. (Tavares-Murta BM et al.: Failure of neutrophil chemotactic function in septic patients. Crit Care Med 30:1056-1061, 2002) eine reduzierte Chemotaxis von PMN in Blut von Sepsispatienten beschrieben. Stephan et al. (Stephan F et al.: Impairment of polymorphonuclear neutrophil functions precedes nosocomial infections in critically illpatients. Crit Care Med 30:315-322, 2002) fanden eine reduzierte Phagozytoseleistung in PMNs von Sepsispatienten und auch Holzer et al. (Holzer K. et al: Phagocytosis by emigrated, intra-abdominal neutrophils is depressed during human secondary peritonitis. Eur Surg Res 34:275-284, 2002.) beobachteten eine langanhaltende Reduktion der Phagozytoseleistung von PMNs von Patienten mit Peritonitis.

Dieses "PMNs Dysfunktionssyndrom" ist mit einer erhöhten Produktion von zytotoxischen freien Sauerstoff und H2O2 kombiniert (Wittmann et al.: Differential effects of clindamycin on neutrophils of healthy donors and septic patients. Int Immunopharmacol 4:929-937, 2004).

Ob sich eine PMN-Dysfunktion entwickelt und in welche Richtung ist gegenwärtig nicht voraus bestimmbar. Sie kann bei entsprechender Prädisposition sowohl in eine Überreaktion münden, die zum ARDS und multiplen Organversagen führt oder zur Immundysfunktion mit Infektion und Sepsis.

Ab einem bestimmten Zeitpunkt eines klinischen Geschehens, dass mit einer PMN-Dysfunktion einhergeht, ist es angezeigt, dem Organismus die unterstützende Behandlung zukommen zu lassen, die eine Rückführung der PMN-Funktion zum Normzustand ermöglicht. Das kann prinzipiell geschehen durch pharmakologische Einflussnahme auf die Freisetzung oder Wirkung pro-inflammatorischer Zytokine (z.B. über neutralisierende oder Rezeptor blockierende Antikörper), die Einleitung der Apoptose bei den aktivierten PMNs, den Schutz des betroffenen Gewebes durch Antioxidantien, die Hemmung der Komplementaktivierung und andere, oder aber durch die extrakorporale Entfernung der aktivierten, deregulierten PMNs.
Wegen der kurzen biologischen Halbwertzeit der PMNs ist eine kontinuierliche Entfernung der aktivierten PMNs in Kombination mit pro-inflammatorischen Zytokinen angezeigt, um das Aktivierungspotential kontinuierlich niedrig zu halten.
Fast ausschließlich auf der Basis von in vitro-Untersuchungen existieren unterschiedlichste meistens unspezifisch wirkende Lösungsvarianten, um die Wirkungen verschiedener Komplementfaktoren zu eliminieren, die aber wegen der zu erwartenden Nebenwirkungen kaum unter in vivo Bedingungen getestet werden können (z.B. WO-A-98/34959). Eine Einbeziehung der PMNs in die Aufgabenstellung und Lösungsansätze erfolgte in der Regel nicht.
In ex vivo Verfahren zur Prävention der Komplementaktivierung durch künstliche, extrakorporale Oberflächen (z.B. Oberflächenbeschichtungen) wurde eine unspezifische Komplementinaktivierung erfolgreich durchgeführt. Desweiteren ist aus US 5,853,722 die selektive Entfernung aktivierter Komplementfaktoren unter Nutzung von spezifischen C5 Antikörpern bekannt und sicher auch zu bevorzugen, zumal hochaffine Antikörper zwischenzeitlich gegen alle Komponenten des Komplementsystems generiert wurden.
Die aufgezeigte funktionelle Kaskade dient vornehmlich der Eliminierung der in den Organismus eingedrungenen Bakterien. Sobald jedoch eine Diskrepanz zwischen der Anzahl und/oder Virulenz der eingedrungenen Bakterien und der Eliminierungskapazität des Immunsystems (z.B. beim posttraumatischen Immundefizit) auftritt, wird eine überschießende Aktivierung beobachtet, die nachfolgend von einer massenhaften Freisetzung von "Schockmediatoren" (Interleukine, Thrombozytenaktivierunsfaktor (PAF), aber auch Sauerstoffradikale, Prostaglandine und deren Stoffwechselprodukte) begleitet wird, die die Eliminierungskapazität weiter einschränkt. Zusätzlich werden auch CD14-negative Zellen (z.B. Endothelien) aktiviert, da im Blutplasma lösliches CD14 (sCD14) als LPS Fänger vorhanden ist, das die Bindung an diese Zellen erleichtert und die Bildung und Freisetzung weiterer Schockmediatoren induziert, die dadurch den Circulus vitiosus verstärken. Da die Schockmediatoren zwar selektiv, aber nicht spezifisch wirken, werden Funktionseinschränkungen in verschiedenen Zellen und Organen beobachtet (PMNs, Lymphozyten, Blutgerinnungssystem, Kreislauf, Komplement-System), so dass die den gesamten Organismus befallenen Entzündungsreaktionen die Schockgenese einleiten, die zu irreversiblen Organschäden, zum Kreislaufzusammenbruch und zum Tod führen.
Um diese Funktionskette zu durchbrechen sind unterschiedliche Therapiestrategien untersucht worden. Die Unterbrechung der Kaskade mit Antikörpern, die die LPS-Bindung an Proteine (LBP, sCD14), an den Rezeptor (CD14), an freigesetzte Zytokine oder an Zytokinrezeptoren unterbrechen bzw. mit Antagonisten, die die funktionellen Bereiche der Rezeptoren blockieren, erbrachten an verschiedenen tierexperimentellen Sepsismodellen zwar beeindruckende Erfolge, jedoch bis heute liegen keine klinisch erprobten, erfolgreichen Präventions- und/oder Therapiestudien vor.
Schefold et al. (Immunadsorption of endotoxin, IL6 and C5a reverses immunparalysis and improves organ function in patients with severe sepsis and septic shock, Shock 2007; 28:418-425) entfernten mittels Immunadsorption Endotoxin, IL6 und C5a. Im Ergebnis konnte die Monozytenfuktion der immunparalysierten Patienten signifikant verbessert werden. Die PMN-Funktion wurde nicht erfasst. Ein Einfluss der Immunadsorption auf die Leukozytenzahl konnte nicht nachgewiesen werden. Versuche die pro-inflammatorischen Zytokine durch eine extra-korporale Blutreinigung zu entfernen und so das aus dem Gleichgewicht geratene Immunsystem wieder zur Normreaktion zurückzuführen sprechen also dafür, dass eine extra-korporale Intervention der immunologischen Dysfunktion ein Weg der therapeutischen Beeinflussung dieser, häufig lebensbedrohender Zustände seien kann. So konnten Hirano et al. (Modulation of Polymorphonuclear Leukocyte Apoptosis in the Critically III by Removal of Cytokines with Continuous Hemodiafiltration, Blood Purif 2004;22:188-197) nachweisen, dass eine kontinuierliche Hämodiafiltration eine signifikante Absenkung der Zytokine bewirkt (IL6), verbunden mit einer erhöhten Apoptoserate der PMNs.

Während es eine große Anzahl von Berichten zum extrakorporalen Entzug von Mediatoren über unspezifische (Dialyse, Hämodiafiltration, Adsorption an Membranen oder Albumin) oder spezifischen (Immunadsorption) Verfahren und ihren Einfluss auf klinische und immunologische Parameter gibt, beziehen sich die Mitteilungen zur therapeutischen Zellabreicherung ausnahmslos auf unspezifische Adsorptionsverfahren von PMN auf Polymeroberflächen.

Die hochgesteckten Erwartungen konnten einerseits deshalb nicht erfüllt werden, da zunehmend erkannt werden musste, dass die Deregulation des Immunsystems nicht nur die Folge einer LPS-Einwirkung auf Zellen und Gewebe ist, sondern die Ursache-Wirkungs-Beziehungen viel komplexerer Natur sind.
Die Entwicklung des Multiorganversagens ist ein sehr dynamisches Geschehen primär unterschiedlicher Genese, bei dem innerhalb kurzer Zeit unterschiedliche Mediatoren und Zellpopulationen sehr verschiedene Reaktionen bewirken, die nach anfänglich lebenserhaltender Funktion rasch durch Fehlregulation zum Multiorganversagen führen.

Die spezifische Separation von aktivierten PMNs über monoklonale Antikörper gegen CD11b für Forschungszwecke wird durch magnetische Verfahren realisiert. Für eine Anwendung am Menschen und/oder in ein kontinuierliches Verfahren sind sie nicht geeignet.

WO 02/24307 offenbart ein Filtersystem zum kontinuierlichen Entzug aktivierter PMNs aus Blut bestehend aus: a. Molekülen, die aktivierte PMNs erkennen und binden (siehe WO 02/24307, Ansprüche 1, 2, 18, hier: Bindungsreagenzien mit Affinität für CD11/CD18, Mac-1, etc. mit beispielhafter Anwendung bei Sepsispatienten) sowie den Merkmalen d., e. und f. (siehe Seite 18, Zeile 30- Seite 19, Absatz 1, Abb. 8).
WO 02/24307 enthüllt keine Kombination mit Bindungsreagenzien, die PMN-aktivierende Mediatoren und Anaphylatoxine erkennen und binden können.

Aufgabe der Erfindung ist ein Verfahren zu entwickeln, dass in Form eines Medizinproduktes für die extra-korporale Behandlung solcher Erkrankungen eingesetzt werden kann wie :
- **Acute respiratory distress syndrome (ARDS) und multiples Organversagen** nach multiplen Skelettverletzungen, schweren OP-Traumen, Lungenverletzungen, etc.
- **Hemolytic Uremic Syndrome (HUS)**
- **Ischämische bzw. Postischämischer Zustand nach Verletzungen,** Entzündungen, Erfrierungen, Verbrennungen, Infarkten, Operationen, radiologischen oder toxischen Einwirkungen von bzw. auf Magen-Darm, Niere, Pankreas, Leber, Herz, Gehirn, Lunge, Haut und andere Organe und Gewebe.
- **Postischämischer Zustand nach starkem Blutverlust**
- **Sepsis und septischer Schock**
- **Alle weiteren pathologischen Zustände, die mit einer PMN-Dysfunktion einhergehen**

Die Erfindung in ihrer breitesten Form wird in den unabhängigen Ansprüchen definiert:
1. Filtersystem zum kontinuierlichen Entzug aktivierter PMNs ("Polymorphonuclear leukocytes") aus Blut bestehend aus
   a. Molekülen, die aktivierte PMNs erkennen und binden
   b. Molekülen, die PMN-aktivierende Mediatoren erkennen und binden
   c. Molekülen, die C3a und/oder C5a erkennen und binden
   d. Biokompatiblen Oberflächen (Träger), auf denen die Moleküle a., b. und c. kovalent oder adsorptiv gebunden sind
   e. einem Gehäuse zur Aufnahme der aktiven Träger d
   f. Ventilen und Schläuchen.
Bevorzugte Ausführungen werden in den abhängigen Ansprüchen 2-15 beansprucht.
Die Ansprüche 16-18 beziehen sich auf die Verwendung der beanspruchten Filtersysteme und der Anspruch 19 bezieht sich auf ein Verfahren zur Herstellung der Vorrichtungen gemäß der Ansprüche 1-15.

Die Aufgabe der Erfindung wurde durch die Kombination von kontinuierlichem Entzug der aktivierten PMNs und der gleichzeitigen oder zeitnahen Reduktion pro-inflammatorischer, PMNs aktivierender Mediatoren erfüllt. Die Einbeziehung der Reduktion von aktivierten Komplement, vorzugsweise C5a, wird immer dann angezeigt sein, wenn komplementbedingt Organschäden zu erwarten sind. Weiterhin bewirkt die Inaktivierung aktivierten Komplementes innerhalb des Medizinproduktes dessen verbesserte Biokompatibilität.

Auch wenn prinzipiell mittels bekannter Filterverfahren die gesamt PMN-Fraktion extrakorporal reduziert werden kann, da insbesondere am Beginn der Behandlung lebensbedrohlicher Zustände wie septischer Schock nahezu alle PMNs aktiviert sind, ist die erfindungsgemäße gezielte Abreicherung der aktivierten PMNs von Vorteil für die notwendige Rückführung der PMNs zur physiologischen Apoptoserate und des Immunsystems zur Normregulation. Eine systematische Entfernung aller PMNs hätte dann fatale Auswirkungen.
Gemeinsam mit den aktivierten PMNs bzw. zeitnahen werden die PMN aktivierenden pro-inflammatorische Zytokine wie TNF-alpha, IL-1ß, IL-6, IL-8, GM-CSF, Interferon-gamma, vorzugsweise IL-6 aus dem Blut entfernt.

Erfindungsgemäß werden Antikörper verwendet, die vornehmlich aktivierte PMNs erkennen sowie die PMN-aktivierenden Zytokine.
Für die PMN-Bindung werden bevorzugt Antikörper verwendet, die an der extrazellularen Region des CD11b, CD18 oder anderen Regionen des CR3 (CD11b/CD18, Mac-1) binden. Erfindungsgemäß können auch Peptide von diesen eingesetzt werden, die Teil der Antigen-bindenden Antikörperspezifität repräsentieren oder Moleküle oder Untereinheiten der spezifischen CD11b-Liganden (iC3b Fibrinogen, Factor X, FcR II, FcR III, uPAR, CD14, ICAM-1, ICAM-2, ICAM-4, Heparin, Haptoglobin, Kininogen, CD23, Neutrophil inhibitory factor from Ancylostoma C, Filamentous hemagglutinin form Bordetella P, gp63 from Leishmenia D, WI-1 antigen from Blastomyces D).
Der erfindungsgemäße Immunadsorber weist als Trägermaterialien an sich übliche Membranen, Fasern, Hohlfasern oder Partikel aus organischen oder synthetischen biokompatiblen Polymeren auf, so z.B. aus Polystyrolen, Kohlenhydraten, wie z.B. Zellulose- oder Agarosederivate oder aus Acrylaten, wobei die spezifischen Liganden (Antikörper oder Peptide) kovalent an diese gebunden oder über Spacer oder Linker an sie fixiert sind.
Die Herstellung der erfindungsgemäßen Immunadsorber erfolgt durch an sich bekannte Methoden, indem die Antikörper, die gegen CD11b, C5a und IL6 sowie ggf. gegen weitere PMN-aktivierenden Mediatoren gerichtet sind, kovalent oder adsorptiv an die Trägermaterialen aus organischen oder synthetischen Polymeren gekoppelt werden. Die spezifischen Antikörper werden durch an sich bekannte Immunisierung vorzugsweise von Kleinsäugern, wie Mäusen, Ratten oder Kaninchen oder Vögeln, wie z.B. Hühnern, mit den entsprechenden Antigenen hergestellt.
Es können Antikörper jeglichen Ursprungs Anwendung finden. Bevorzugt handelt es sich um monoklonale Antikörper oder polyklonale Antikörper besonders bevorzugt um aviäre Antikörper des Typs IgY. Gemäß der Erfindung handelt es sich dabei um Antikörper, die gegen CD11b, C5a und IL6 gerichtet sind.
Gegenstand der Erfindung ist auch die Verwendung der Adsorber in Vorrichtungen zur Entfernung von aktivierten PMNs, IL-6, Komplementfaktor C5a und ggf. von weiteren Mediatoren aus Blut in Abhängigkeit der patientenspezifischen Situation
Bevorzugt werden die Adsorber in der Therapie von Erkrankungen eingesetzt, die mit einem PMN Dysfunktionssyndrom einhergehen. Es sind aber auch therapeutische Blutbehandlungen möglich, wo aktiviertes Komplement eine prominente pathogenetische Bedeutung für Organschäden haben könnte, wie z.B. bei Hirntraumen, Herzinfarkt oder zerebraler Malaria.
Obwohl für die meisten Substanzen Antikörper verfügbar sind, die nach bekannten Methoden an die unterschiedlichen Träger gekoppelt werden können, werden bevorzugt monoklonale und/oder aviäre Antikörper verwendet. Aviäre Antikörper aktivieren im Gegensatz zu Säuger-Antikörpern das Komplementsystem nicht. Da die aktivierenden Eigenschaften an den F_{c}-Teil der Säuger-Antikörper gebunden sind, kann prinzipiell auch das mit Papain abgespaltene F_{ab}-Fragment verwendet werden bzw. können "humanisierte" monoklonale Antikkörper zur Anwendung kommen.
Nach derzeitigem Kenntnisstand haben immobilisierte aviäre Antikörper keinerlei unspezifische Wirkungen auf das Abwehrsystem des Menschen. Vögel, vorzugsweise Hühner werden mit üblichen Verfahren ohne oder mit Verwendung von Adjuvantien immunisiert. Die spezifischen Immunglobuline werden im Eidotter ausgeschieden und können hieraus mit üblichen Methoden isoliert werden. Sie werden mit bekannten Verfahren kovalent am Fc-Teil an Mikropartikel oder Membranen gebunden.
Mit dem erfindungsgemäßen Adsorptionssystem für die extrakorporale Zellabreicherung steht erstmals ein selektives System zur Verfügung, welches patientenspezifisch einsetzbar ist und womit Fehlregulationen des Immunsystems behoben werden können. Durch die Kombination mit dem Entzug pro-inflammatorischer, PMNs aktivierender Zytokine wird zeitgleich das PMN-Dysfunktionssyndrom behandelt (Rückführung zur normalen Apoptose- und Phagozytoserate) wie auch der systemische Effekt einer überschießenden pro-inflammatorischen Reaktionslage.

Die Regenerationsmöglichkeit der Vorrichtung macht die kontinuierliche Entnahme großer Zellmengen ökonomisch machbar.

## Patentansprüche

1. Filtersystem zum kontinuierlichen Entzug aktivierter PMNs ("Polymorphonuclear Leukocytes") aus Blut bestehend aus
a. Molekülen, die aktivierte PMNs erkennen und binden
b. Molekülen, die PMN-aktivierende Mediatoren erkennen und binden
c. Molekülen, die C3a und/oder C5a erkennen und binden
d. Biokompatiblen Oberflächen (Träger), auf denen die Moleküle a., b. und c. kovalent oder adsorptiv gebunden sind
e. einem Gehäuse zur Aufnahme der aktiven Träger d
f. Ventilen und Schläuchen.

2. Filtersystem nach Anspruch 1, **dadurch gekennzeichnet, dass** die Moleküle a) Polypeptide sind, die eine Aminosäuresequenz natürlichen oder künstlichen Ursprungs enthalten, die spezifisch Oberflächenstrukturen der aktivierten PMN erkennen und binden.

3. Filtersystem nach Anspruch 1, **dadurch gekennzeichnet, dass** die Moleküle b) Polypeptide sind, die eine Aminosäuresequenz natürlichen oder künstlichen Ursprungs enthalten, die PMN-aktivierende Mediatoren erkennen und binden.

4. Filtersystem nach Anspruch 1, **dadurch gekennzeichnet, dass** die Moleküle c) Polypeptide sind, die eine Aminosäuresequenz natürlichen oder künstlichen Ursprungs enthalten, die C3a und/oder C5a erkennen und binden.

5. Filtersystem nach den Ansprüchen 2-4, **dadurch gekennzeichnet, dass** die Polypeptide Antikörper jeglichen Ursprungs sind.

6. Filtersystem nach den Ansprüchen 2-5, **dadurch gekennzeichnet, dass** die Polypeptide monoklonale und / oder polyklonale Antikörper sind.

7. Filtersystem nach Anspruch 6, **dadurch gekennzeichnet, dass** die polyklonalen Antikörper aviären Ursprung sind.

8. Filtersystem nach Anspruch 3, **dadurch gekennzeichnet, dass** die Mediatoren TNF-alpha, IL-1ß, IL-6, IL-8, GM-CSF, Interferon-gamma sind.

9. Filtersystem nach Anspruch 8, **dadurch gekennzeichnet, dass** IL-6 erkannt und gebunden wird.

10. Filtersystem nach Anspruch 2, **dadurch gekennzeichnet, dass** Teile des CD11 b/CD18 erkannt und gebunden werden.

11. Filtersystem nach den Ansprüchen 1-10, **dadurch gekennzeichnet, dass** die biokompatiblen Träger alle Formen haben, vorzugsweise aber Partikel verwendet werden.

12. Filtersystem nach Anspruch 1-11, **dadurch gekennzeichnet, dass** Partikel mit einem Durchmesser zwischen 10-1000µm eingesetzt werden.

13. Filtersystem nach den Ansprüchen 1-12, **dadurch gekennzeichnet, dass** das Gehäuse so gestaltet ist, das es eine Ablösung der Mediatoren und Zellen zur Regenerierung der Partikel innerhalb oder außerhalb der Vorrichtung erlaubt.

14. Filtersystem nach den Ansprüchen 1-13, **dadurch gekennzeichnet, dass** sie mittels Schläuchen, Ventilen und Pumpen Bestandteil eines extra-korporalen Blutkreislaufes ist.

15. Filtersystem nach den Ansprüchen 1-14, **dadurch gekennzeichnet, dass** sie aus mindestens 2 Einheiten besteht, so dass eine kontinuierliche Blutbehandlung möglich ist.

16. Filtersystem gemäß einem der Ansprüche 1-15 zur Verwendung für die Behandlung krankhafter Zustände von den Individuen, die mit einer Fehlfunktion von PMNs einhergeht.

17. Filtersystem gemäß einem der Ansprüche 1-16 für die Verwendung zur Behandlung von Acute respiratory distress syndrome (ARDS), multiplem Organversagen, Hemolytic Uremic Syndrome (HUS), ischämischem bzw. postischämischem Zustand nach Verletzungen, Entzündungen, Erfrierungen, Verbrennungen, Infarkten, Operationen, radiologischen oder toxischen Einwirkungen von bzw. auf Organe und Gewebe, postischämischer Zustand nach starken Blutverlust, Sepsis und/ oder septischem Schock.

18. Filtersystem gemäß einem der Ansprüche 1-15 für die Vervendung zur Behandlung von Erkrankungen, bei denen aktiviertes Komplement eine prominente pathogenetische Bedeutung für Organschäden haben könnte, wie z.B. bei Hirntraumen, Herzinfarkt oder zerebraler Malaria.

19. Verfahren zur Herstellung einer Vorrichtung gemäß einem der Ansprüche 1-15.

## Claims

1. Filter system for the continuous removal of activated PMNs ("Polymorphnuclear Leucocytes") from blood, comprises of
a. Molecules identifying and binding activated PMNs
b. Molecules identifying and binding PMN-activating mediators
c. Molecules identifying and binding C3a and/or C5a
d. Biocompatible surfaces (carrier) on which the molecules a., b., and c. are covalently or adoptively bound
e. A housing to carry the activated carrier d
f. Valves and tubes.

2. Filter system according to claim 1, **characterized in that** molecules a), which are polypeptides containing an amino acid sequence of natural or artificial origin, which identify and bind on specific structures of the surface of activated PMN

3. Filter system according to claim 1, **characterized in that** molecules b), which are polypeptides containing an amino acid sequence of natural or artificial origin, which identify and bind PMN-activating mediators.

4. Filter system according to claim 1, **characterized in that** molecules c), which are polypeptides containing an amino acid sequence of natural or artificial origin, which identify and bind C3a and/or C5a.

5. Filter system according to claims 2-4, **characterized in that** the polypeptides are antibodies of any origin.

6. Filter system according to claims 2-5, **characterized in that** the antibodies are monoclonal and/or polyclonal.

7. Filter system according to claim 6, **characterized in that** the polyclonal antibodies are of avian origin.

8. Filter system according to claim 3, **characterized in that** the mediators are TNFα, IL-1ß, IL-6, IL-8, GM-CSF, and interferon-gamma.

9. Filter system according to claim 8, **characterized in that** IL-6 is identified and bound.

10. Filter system according to claim 2, **characterized in that** parts of CD11b/CD18 are identified and bound.

11. Filter system according to claims 1-10, **characterized in that** biocompatible carrier of all shapes, but preferably particle, are used.

12. Filter system according to claims 1-11, **characterized in that** particle with a diameter between 10-1000 µm are used

13. Filter system according to claims 1-12, **characterized in that** the housing is formed in a way which allows the detachment of mediators and cells within or outside of the device for the purpose of regeneration.

14. Filter system according to claims 1-13, **characterized in that** it is part of a extra-corporeal circuit by means of tubes, valves, and pumps.

15. Filter system according to claims 1-14, **characterized in that** at least two units are used, so that a continuous blood treatment is possible.

16. Filter system according to one of the claims 1-15 for usage to treat of pathologic situations in individuals which are combined with a dysfunction of PMNs.

17. Filter system according to one of the claims 1-16 for usage to treat of the Acute Respiratory Distress Syndrome (ARDS), multi-organ failure, Hemolytic Uremic Syndrome (HUS), Ischemic or post-ischemic conditions after injuries, inflammations, frostbites, burnings, infarcts, operations, radiologic or toxic action on or to organs and tissues, posf-ischemic conditions after severe loss of blood, sepsis and/ or septic shock

18. Filter system according to one of the claims 1-15 for usage for treatment of diseases in which activated complement has a prominent role in the pathogenesis of organ damages like brain trauma, heart infarct or cerebral malaria.

19. Procedure for manufacturing of a device according to claims 1-15.

## Revendications

1. Système de filtration pour le retrait continu de LPN (leucocytes polymorphonucléaires) activés du sang, constitué de
a. molécules qui reconnaissent et lient les LPN activés
b. molécules qui reconnaissent et lient les médiateurs activant les LPN
c. molécules qui reconnaissent et lient les C3a et/ou C5a
d. surfaces (supports) biocompatibles sur lesquelles les molécules a., b. et c. sont liées par covalence ou adsorption
e. un boîtier destiné à recevoir les supports actifs d.
f. valves et tuyaux.

2. Système de filtration selon revendication 1, **caractérisé en ce que** les molécules a) sont des polypeptides qui contiennent une séquence d'acides aminés d'origine naturelle ou artificielle et qui reconnaissent et lient spécifiquement les structures des surfaces des LPN activés.

3. Système de filtration selon revendication 1, **caractérisé en ce que** les molécules b) sont des polypeptides qui contiennent une séquence d'acides aminés d'origine naturelle ou artificielle et qui reconnaissent et lient les médiateurs activant les LPN.

4. Système de filtration selon revendication 1, **caractérisé en ce que** les molécules c) sont des polypeptides qui contiennent une séquence d'acides aminés d'origine naturelle ou artificielle et qui reconnaissent et lient les C3a et/ou C5a.

5. Système de filtration selon revendications 2 à 4, **caractérisé en ce que** les polypeptides sont des anticorps de toute origine.

6. Système de filtration selon revendications 2 à 5, **caractérisé en ce que** les polypeptides sont des anticorps monoclonaux et/ou polyclonaux.

7. Système de filtration selon revendication 6, **caractérisé en ce que** les anticorps polyclonaux sont d'origine aviaire.

8. Système de filtration selon revendication 3, **caractérisé en ce que** les médiateurs sont des TNF-alpha, IL-1beta, IL-6, IL-8, GM-CSF, interférons gamma.

9. Système de filtration selon revendication 8, **caractérisé en ce que** IL-6 est reconnu et lié.

10. Système de filtration selon revendication 2, **caractérisé en ce que** des parties du CD11 b/CD18 sont reconnues et liées.

11. Système de filtration selon revendications 1 à 10, **caractérisé en ce que** les supports biocompatibles ont toutes les formes, mais que ce sont de préférence des particules qui sont utilisées.

12. Système de filtration selon revendications 1 à 11, **caractérisé en ce que** des particules d'un diamètre entre 10-1000 µm sont utilisées.

13. Système de filtration selon revendications 1 à 12, **caractérisé en ce que** le boîtier est conçu de sorte à permettre une séparation des médiateurs et des cellules en vue de la régénération des particules à l'intérieur ou à l'extérieur du dispositif.

14. Système de filtration selon revendications 1 à 13, **caractérisé en ce qu'**il est par le biais de tuyaux, valves et pompes un élément d'un circuit sanguin extra-corporel.

15. Système de filtration selon revendications 1 à 14, **caractérisé en ce qu'**il est composé de deux unités au moins, de sorte à permettre un traitement sanguin en continu.

16. Système de filtration selon l'une des revendications 1 à 15 destiné à l'utilisation pour le traitement d'états maladifs d'individus souffrant d'un dysfonctionnement de LPN.

17. Système de filtration selon l'une des revendications 1 à 16 destiné à l'utilisation pour le traitement du syndrome de détresse respiratoire aiguë (SDRA), de la défaillance organique multiple, du syndrome hémolytique et urémique (SHU), de l'état ischémique et post-ischémique après blessures, inflammations, gelures, brûlures, infarctus, opérations, effets radiologiques ou toxiques des ou sur les organes et les tissus, de l'état post-ischémique après une forte perte de sang, une septicémie et/ou un choc septique.

18. Système de filtration selon l'une des revendications 1 à 15 destiné à l'utilisation pour le traitement de maladies lors desquelles un complément activé pourrait jouer un rôle pathogénique essentiel pour les dommages organiques, par exemple en cas de lésions cérébrales, d'infarctus du myocarde ou de malaria cérébrale.

19. Procédé destiné à la réalisation d'un dispositif selon l'une des revendications 1 à 15.
